(19) Europäisches Patentamt
European Patent Office
Office européen des brevets

(11) **EP 3 729 074 B1**

(12) **EUROPÄISCHE PATENTSCHRIFT**

(45) Veröffentlichungstag und Bekanntmachung des Hinweises auf die Patenterteilung:
**23.11.2022 Patentblatt 2022/47**

(21) Anmeldenummer: **18822022.2**

(22) Anmeldetag: **10.12.2018**

(51) Internationale Patentklassifikation (IPC):
**G01N 33/06** $^{(2006.01)}$        **G01N 22/00** $^{(2006.01)}$

(52) Gemeinsame Patentklassifikation (CPC):
**G01N 33/06; A01J 5/0133; A01J 5/0138; G01F 1/74; G01F 1/8436; G01F 15/02; G01N 9/26; G01N 9/32; G01N 9/36; G01N 22/00**

(86) Internationale Anmeldenummer:
**PCT/EP2018/084185**

(87) Internationale Veröffentlichungsnummer:
**WO 2019/121109 (27.06.2019 Gazette 2019/26)**

(54) **VERFAHREN UND VORRICHTUNG ZUR MILCHFETTMESSUNG**

METHOD AND DEVICE FOR MEASURING FAT IN MILK

PROCÉDÉ ET DISPOSITIF DE MESURE DE LA MATIÈRE GRASSE DU LAIT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2017 DE 102017131269**

(43) Veröffentlichungstag der Anmeldung:
**28.10.2020 Patentblatt 2020/44**

(73) Patentinhaber: **Endress + Hauser Flowtec AG 4153 Reinach (CH)**

(72) Erfinder:
• **PFLÜGER, Stefan 80995 München (DE)**

• **DRAHM, Wolfgang 85435 Erding (DE)**
• **ZHU, Hao 85354 Freising (DE)**

(74) Vertreter: **Penner, Paul Endress + Hauser Group Services (Deutschland) AG+Co. KG PatServe Colmarer Straße 6 79576 Weil am Rhein (DE)**

(56) Entgegenhaltungen:
**US-A- 5 103 181          US-A- 6 147 502 US-A1- 2016 313 259**

**Beschreibung**

Die vorliegende Erfindung betrifft ein Verfahren und eine Vorrichtung zur Milchfettmessung:
Milch und daraus gewonnene (Zwischen-)Produkte lassen sich als Mischung verschiedener Komponenten beschreiben, die hauptsächlich aus Wasser, Milchfett und anderen Feststoffen bestehen, wobei die anderen Feststoffe im Wesentlichen Proteine, Kohlenhydrate (darunter insbesondere Lactose) und in geringen Mengen Mineralstoffe umfassen.

Während der Verarbeitungskette beginnend bei der Rohmilch bis zum fertigen Milchprodukt sind die Anteile dieser Komponenten wichtige Parameter für die Steuerung und Regelung der Prozesse, die Prozess- und Qualitätskontrolle, und die Bilanzierung von Produktströmen. Es ist üblich, die Anteile durch Laborproben nach Standardverfahren zu bestimmen. Dies bedingt, dass nur wenige Proben ausgewertet können, und dass ein Analyseergebnis erst deutlich verzögert nach der Probenahme vorliegt.

Eine prozessnahe spektroskopische Analyse im Infrarot-Bereich mit automatisierter Probenahme ist zwar möglich, aber diese ist erstens sehr kostenintensiv und zweitens nur auf das geringe Volumen der in vergleichsweise großen Zeitintervallen genommenen Proben gestützt. Zur Prozesssteuerung ist eine solche Analyse daher nur bedingt geeignet.

Eine Dichtemessung, z.B. mittels Coriolis-Durchflussmesser, ist zwar als Inline-Messung zur kontinuierlichen Prozessüberwachung geeignet, und kann auch zur Bestimmung des Fettgehaltes bzw. des Feststoffanteils von Milch verwendet werden, wenn die Annahme eines gegebenen Verhältnisses der Feststoffanteile von Fett, Kohlenhydraten (insbesondere Lactose), Proteinen usw. gerechtfertigt ist. Mit abnehmender Gültigkeit dieser Annahme wird das Messergebnis ungenau.

Eine weitere Schwierigkeit bei der Bestimmung des Fettgehaltes mit einem Coriolis-Durchflussmesser ergibt sich durch in der Milch verteilte Mikroblasen von Luft, die einerseits die effektive Dichte verringern und andererseits aufgrund von Schwingungen der nunmehr kompressiblen Milch gegenüber dem Messrohr zu veränderten Beziehungen zwischen Eigenfrequenzen der schwingenden Messrohre des Durchflussmessers und der Dichte des Messstoffs in den Messrohren führen. Die eingeschränkte Eignung von Coriolis-Massedurchflussmessern für die Bestimmung der Zusammensetzung mit Gas beladener Flüssigkeiten ist beispielsweise in US 7,363,800 B2 beschrieben. Demnach lehrt dieser Stand der Technik eine Anordnung mit erstens einem Mikrowellensensor zum Ermitteln dielektrischer Parameter eines Mediums, zweitens einem Coriolis-Massedurchflussmesser, drittens einem unabhängigen Sensor zur Bestimmung des Gasanteils des Mediums und viertens eine Signalverarbeitungseinheit zur Verarbeitung der Signale der verschiedenen Sensoren. Dies ist jedoch eine aufwändige und kostenintensive Vorrichtung.

Eine Messanordnung zur Bestimmung des Fettanteils von Milch, umfassend einen Dichtemesser, einen Drucksensor und einen Mikrowellensensor, wird in US 5,103,181 A offenbart.

Es ist daher die Aufgabe der vorliegenden Erfindung ein Verfahren und eine entsprechende Messanordnung bereitzustellen zur zuverlässigen, kontinuierlichen Messung des Fettanteils von Milch auch bei veränderlichen Feststoffanteilen und veränderlicher Gasbeladung.

Die Aufgabe wird erfindungsgemäß gelöst durch das Verfahren gemäß dem unabhängigen Patentanspruch 1 und die Messanordnung gemäß dem unabhängigen Patentanspruch 8.

Das erfindungsgemäße Verfahren zum kontinuierlichen Bestimmen des Fettgehaltes von Milch mit veränderlichen Feststoffanteilen, welche mit veränderlicher Gasbeladung in einer Rohrleitung fließt, umfasst:

Ermitteln eines Wertes für die Schallgeschwindigkeit und eines mittleren Dichtewertes für die in der Rohrleitung strömende Milch auf Basis der Eigenfrequenzen mindestens zweier Biegeschwingungsnutzmoden von Messrohren eines in der Rohrleitung angeordneten Dichtemessers;

Ermitteln eines Wertes für den statischen Druck in der Rohrleitung mittels eines an die Rohrleitung angeschlossenen Drucksensors;

Ermitteln eines Werts für den Gasvolumenanteil auf Basis des Werts für die Schallgeschwindigkeit, des Werts für die mittlere Dichte und des Werts für den Druck;

Ermitteln eines Werts der Dichte der in der Rohrleitung strömenden Milch ohne die Gasbeladung auf Basis des Werts für die mittlere Dichte und auf Basis des Werts für den Gasvolumenanteil;

Ermitteln eines Werts für die Permittivität der in der Rohrleitung strömenden Milch auf Basis von mindestens einer Messung der Ausbreitungsgeschwindigkeit und/oder Absorption von Mikrowellen in der Milch mittels eines in der Rohrleitung angeordneten Mikrowellensensors; und

Berechnen des Fettanteils auf Basis des Werts der Dichte der in der Rohrleitung strömenden Milch ohne die Gasbeladung und des Werts für die effektive Permittivität.

In einer Weiterbildung der Erfindung wird die Milch als Dreikomponentensystem modelliert, wobei die Komponenten Fett, Wasser und fettfreie Feststoffe umfassen.

In einer Weiterbildung der Erfindung umfassen die Feststoffe Proteine und Kohlenhydrate, darunter insbesondere Lactose.

In einer Weiterbildung der Erfindung ist die Dichte der in der Rohrleitung strömenden Milch ohne die Gasbeladung als Funktion, beispielsweise als lineare Funktion der Konzentration der in der Milch enthaltenen Komponenten modelliert mit den Dichtewerten der reinen Komponenten als Gewichtungsfaktoren; wobei die effektive Permittivität der in der Rohrleitung strömenden Milch unter Berücksichtigung der Gasbeladung als Funktion der Konzentration der in der Milch enthaltenen Komponenten und der Permittivitätswerte der reinen Komponenten modelliert wird; und wobei die Konzentration der Komponenten ermittelt wird, die zu den ermittelten Werten der Dichte und der effektiven Permittivität der Milch führen.

In einer Weiterbildung der Erfindung erfolgt die Bestimmung der Permittivität bei mindestens einer Frequenz oberhalb von 1 GHz, insbesondere oberhalb von 2 GHz, beispielsweise bei 2,45 GHz.

In einer Weiterbildung der Erfindung umfasst das Verfahren weiterhin das Messen der Temperatur der in der Rohrleitung strömenden Milch; und das Ermitteln von temperaturabhängigen Werten für die Dichte und/oder Permittivität der in der Milch enthaltenen Komponenten.

In einer Weiterbildung der Erfindung umfasst der Dichtemesser ein Coriolis-Massedurchflussmessgerät, wobei das Verfahren weiterhin umfasst:

Ermitteln des Massedurchflusses, des Volumendurchflusses und/oder des Fettdurchflusses in der Rohrleitung.

Die erfindungsgemäße Messanordnung, eingerichtet zum Bestimmen des Fettgehaltes von Milch in einer Rohrleitung mit dem oben beschriebenen Verfahren umfasst:

einen Dichtemesser mit mindestens einem schwingfähigen Messrohr, eingerichtet zum Ermitteln eines Dichtemesswertes und eines Schallgeschwindigkeitsmesswertes eines in dem Messrohr enthaltenen Mediums auf Basis mindestens von Nutzmodeeigenfrequenzen mindestens zweier Biegeschwingungsnutzmoden;

einen Drucksensor, eingerichtet zum Messen eines absoluten Drucks eines Mediums;

einen Mikrowellensensor, eingerichtet zum Ermitteln der Absorption und/oder Ausbreitungsgeschwindigkeit von Mikrowellensignalen in einem Medium;

und eine Rechnereinheit, eingerichtet zum Berechnen des Fettgehaltes auf Basis der Messwerte des Dichtemessers, des Drucksensors und des Mikrowellensensors.

In einer Weiterbildung der Erfindung umfasst die Messanordnung außerdem die Rohrleitung, wobei der Dichtemesser, der Drucksensor und der Mikrowellensensor in der Rohrleitung eingebaut sind.

In einer Weiterbildung der Erfindung umfasst der Dichtemesser einen Coriolis Massedurchflussmesser.

Die Erfindung wird nun anhand des in den Zeichnungen dargestellten Ausführungsbeispiels näher erläutert. Es zeigt:

Fig. 1: ein Flussdiagramm eines Ausführungsbeispiels des erfindungsgemäßen Verfahrens;

Fig. 2: ein detaillierteres Flussdiagramm eines Ausführungsbeispiels eines ersten Teilprozesses des erfindungsgemäßen Verfahrens;

Fig. 3: ein detaillierteres Flussdiagramm eines Ausführungsbeispiels eines zweiten Teilprozesses des erfindungsgemäßen Verfahrens; und

Fig. 4: eine schematische Darstellung eines Ausführungsbeispiels einer erfindungsgemäßen Vorrichtung.

Die Komponenten von Milch können im Wesentlichen in vier Gruppen zusammengefasst werden, nämlich Wasser, Fett, Protein und Kohlenhydrate, wobei letztere beispielsweise zu mehr als 95% Lactose und in geringen Anteilen Glucose und Galactose umfassen. Zudem können in Abhängigkeit von physikalischen Prozessbedingungen, insbesondere in Abhängigkeit von vorliegenden Fließbedingungen Lufteinschlüsse in Form von Mikroblasen vorliegen, die bei der Analyse zu berücksichtigen sind. In der folgenden Tabelle sind exemplarisch die physikalischen Eigenschaften der Komponenten und Luft aufgelistet:

|  | Dichte $\rho$ [g/cm$^2$] @ 20°C | Relative Permittivität $\varepsilon$'(f=2.45GHz) @ 20°C |
|---|---|---|
| Wasser | 0.998 [1] | 78 [2] |
| Fett | 0.931 [1] | 2.6 [2] |
| Protein | 1.451 [1] | 1.6 [2] |
| Kohlehydrate (Lactose) | 1.545 [1] | 1.9 [2] |

(fortgesetzt)

|  | Dichte $\rho$ [g/cm$^2$] @ 20°C | Relative Permittivität $\varepsilon'$(f=2.45GHz) @ 20°C |
|---|---|---|
| Luft | 0.0012 | 1.0 |

Mit Hilfe dieser Größen kann die effektive Dichte und Permittivität der Mischung jeweils als Funktion der Anteile ai der Komponenten angegeben werden:

$$\rho_{\text{Milch mit Luft}} = f((1- a_{Luft}) a_{Wasser}, (1- a_{Luft}) a_{Fett}, (1- a_{Luft}) a_{SNF}, a_{Luft})$$

$$\varepsilon'_{eff} = f((1- a_{Luft}) a_{Wasser}, (1- a_{Luft}) a_{Fett}, (1- a_{Luft}) a_{SNF}, \alpha)$$

Der Luftanteil $a_{Luft}$ kann mittels des Dichtemessers und der Hilfsgröße Druck ermittelt werden und ist in den Gleichungen nicht mehr als unbekannter Parameter zu berücksichtigen. Ebenso verhält es sich natürlich mit Prozessen, in denen Luftanteile prozessbedingt nicht vorkommen können ($a_{Luft}$=0).

Da sowohl die Dichte als auch die Permittivität von Kohlenhydraten und Proteinen nahezu identisch sind, können diese ohne wesentlichen Fehlereinfluss zu einer Komponente fettfreie Feststoffe (bzw. Solids-NonFat (SNF)) zusammengefasst werden und mit einer mittleren Dichte und Permittivität rechnerisch berücksichtigt werden, die sich unter Berücksichtigung des typischen Mischungsverhältnisses der beiden Komponenten in Milch ergibt. Bei Kuhmilch wären es lt. Wikipedia rund 58% Kohlenhydrate (96% davon Lactose) und 42% Proteine.

Die mittlere Dichte kann im Wesentlichen als gewichtetes Mittel der Einzeldichten beschrieben werden.

Eine typische Mischungsgleichung, um $\varepsilon'_{eff}$ in einer Mischung mit mehreren Komponenten zu bestimmen, ist die Bruggemann-Formel:

$$\frac{\epsilon_{MG} - \epsilon_h}{\epsilon_{MG} + 2\epsilon_h} = \sum_{n=1}^{N} f_n \frac{\epsilon_n - \epsilon_h}{\epsilon_n + 2\epsilon_h}$$

Dabei sind:

$\epsilon_{MG}$: $\varepsilon'_{eff}$
$\epsilon_h$: Permittivität der durchgehenden Phase (Wasser)
$\epsilon_n$: Permittivität der Zuschlagstoffe (Fett, SNF, Luft)
$f_n$: Volumenanteile der jeweiligen Komponente

[Formel übernommen aus V. Markel - Introduction to the Maxwell Garnett Approximation, Journal of the Optical Society of America A]

Es ergibt sich:

$$\rho_{Milch} = f(a_{Wasser}, a_{Fett}, a_{SNF}) \qquad (G1)$$

$$\varepsilon'_{eff} = f((1- a_{Luft}) a_{Wasser}, (1- a_{Luft}) a_{Fett}, (1- a_{Luft}) a_{SNF}, a_{Luft}) \qquad (G2)$$

Wobei hier $a_{Luft}$ den Volumenanteil der Gasbeladung beschreibt.

Eine dritte Gleichung ergibt sich aus der Summe der Volumenanteile:

$$a_{Wasser} + a_{Fett} + a_{SNF} = 1 \qquad (G3)$$

Es verbleiben im Ergebnis drei Gleichungen mit drei Unbekannten, mit denen die Bestimmung der Anteile von Wasser, Fett, und fettfreien Feststoffen ohne weitere Annahmen möglich ist.

In der Praxis sind Dichte und Permittivität von der Temperatur und die Permittivität von der Messfrequenz abhängig. Eine Temperaturmessung zur Berücksichtigung der Temperaturabhängigkeiten der Stoffeigenschaften bei der Lösung des obigen Gleichungssystems ermöglicht eine gewünschte Genauigkeit.

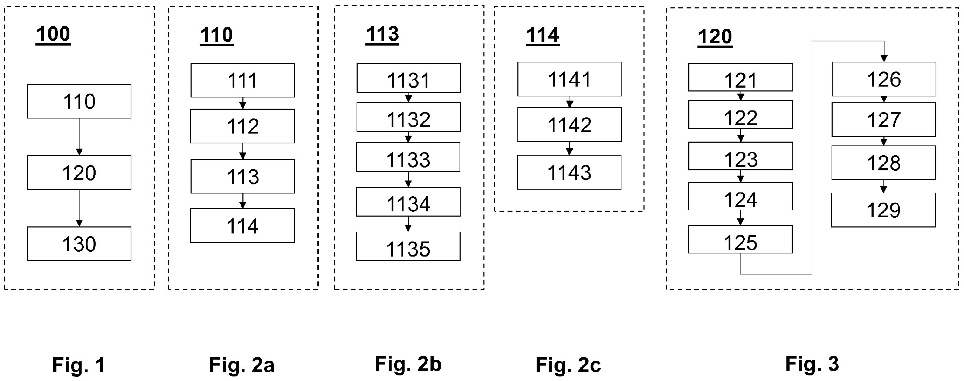

Wie in Fig. 1 dargestellt, beginnt das Verfahren 100 mit einem ersten Teilprozess 110 zum Bestimmen der Dichte der von Luftanteilen befreiten Milch $\rho_{Milch}$. Dies geschieht über die Ermittlung der mittleren Dichte, also die Dichte der mit Luft beladenen Milch $\rho_{Milch\ mit\ Luft}$, Ermittlung des Luftanteils $a_{Luft}$ und Korrektur der Dichte um den Luftanteil.

In einem zweiten Teilprozess 120 folgt die Bestimmung der effektiven Permittivität $\varepsilon'_{eff}$, was über die Messung der Ausbreitungseigenschaften einer elektromagnetischen Welle in der Milch erfolgt.

In einem dritten Teilprozess 130 wird das Gleichungssystem G1, G2, G3 gelöst, um den Fettanteil und ggf. die Anteile anderer Komponenten zu bestimmen.

Wie in Fig. 2a dargestellt, sind im zweiten Teilprozess 110 im Einzelnen die folgenden Schritte durchzuführen: In einem Schritt 111 erfolgt die Bestimmung der Eigenfrequenzen der $f_1$-Biegeschwingungsmode und der $f_3$-Biegeschwingungsmode eines Coriolis-Massedurchflussmessaufnehmers, der hier auch zur Dichtemessung eingesetzt wird. Hierzu können die $f_1$-Biegeschwingungsmode und der $f_3$-Biegeschwingungsmode insbesondere gleichzeitig angeregt werden. Durch Maximieren des Verhältnisses von der Schwingungsamplitude zur modenspezifischen Erregerleistung durch Variieren der Anregungsfrequenzen können die gesuchten Eigenfrequenzen ermittelt werden.

Anhand der ermittelten Eigenfrequenzen $f_i$ werden in einem Schritt 112 vorläufige Dichtewerte $\rho_1$ und $\rho_3$ bestimmt als:

$$\rho_i = c_{0i} + c_{1i}\frac{1}{f_i^2} + c_{2i}\frac{1}{f_i^4}\,,$$

wobei $c_{0i}$, $c_{1i}$, und $c_{2i}$, modenabhängige Koeffizienten sind.

In einem Schritt 113, erfolgt die Bestimmung der Schallgeschwindigkeit der mit Gas beladenen Flüssigkeit und ggf. eines Korrekturterms für die Dichtemessung.

Anschließend wird in einem Schritt 114 mittels der Schallgeschwindigkeit und einem Druckmesswert ein Gasvolumenanteil $a_{Luft}$ berechnet, und die Dichte der von der Luft bereinigten Milch wird berechnet, wie weiter unten näher erläutert wird.

Wie in Fig. 2b dargestellt, umfasst der Schritt 113 zum Bestimmen des Korrekturterms zunächst in einem Schritt 1131 das Berechnen des Verhältnisses V der vorläufigen Dichtewerte, also beispielsweise die Division der vorläufigen Dichtewerte $\rho_1$ und $\rho_3$ zu $V := \rho_1 / \rho_3$.

Anschließend wird in einem Schritt 1132 ein Wert der Schallgeschwindigkeit c bestimmt, welcher mit den gemessenen Eigenfrequenzen $f_1$ und $f_3$ der Biegeschwingungsmoden in der folgenden Gleichung zu dem beobachteten Verhältnis V der vorläufigen Dichtewerte führt:

$$\frac{\left(1 + \dfrac{r}{\left(\dfrac{g \cdot c}{f_1}\right)^2 - b}\right)}{\left(1 + \dfrac{r}{\left(\dfrac{g \cdot c}{f_3}\right)^2 - b}\right)} = V$$

wobei r etwa 0,84, b=1 und g ein messrohrabhängiger Proportionalitätsfaktor zwischen Schallgeschwindigkeit und Resonanzfrequenz ist, der beispielsweise einen Wert von 10/m annehmen kann. Der Wert der Schallgeschwindigkeit, welcher die obige Gleichung erfüllt, ist der gesuchte Wert für die Schallgeschwindigkeit der mit Gas beladenen Flüssigkeit.

Anhand des ermittelten Schallgeschwindigkeitswerts kann dann im Schritt 1133 des Verfahrens in Fig. 2 ein modenspezifischer Korrekturterm Ki für den Resonatoreffekt berechnet werden gemäß:

$$K_i := \left(1 + \frac{r}{\left(\frac{g \cdot c}{f_i}\right)^2 - 1}\right).$$

Ein Dichtewert für die mit Luft beladene Milch $\rho_{Milch\ mit\ Luft}$ kann schließlich im Schritt 1134 berechnet werden als:

$$\rho_{Milch\ mit\ Luft} := \frac{\rho_i}{K_i} \qquad (M1)$$

Die Bestimmung des Luftanteils und die Berechnung der Dichte der von der Luft befreiten Milch in Schritt 114 ist in Fig. 2c näher dargestellt und beruht auf dem folgenden Zusammenhang zwischen der Schallgeschwindigkeit einer mit Gas beladenen Flüssigkeit und weiteren Parametern:

$$c = \left[\frac{\alpha}{c_{Luft}{}^2} + \frac{(1-a_{Luft})^2}{c_{Milch}{}^2} + \frac{\alpha(1-a_{Luft})\cdot\rho_{Milch}}{\gamma\cdot p}\right]^{-\frac{1}{2}} \qquad (C1)$$

Hierbei sind $a_{Luft}$ der Luftvolumenanteil, $c_{Luft}$ die Schallgeschwindigkeit in Luft, $c_{Milch}$ die Schallgeschwindigkeit in Milch ohne Luft $\gamma$ der Adiabatenkoeffizient für Luft, p der aktuelle Druck der mit Luft beladenen Milch und $\rho_{Milch}$ die Dichte der Milch ohne Luft Flüssigkeit.

Die Dichte von der mit Luft beladenen Milch ergibt sich als gewichtete Summe der Einzeldichten. Insofern, als die Dichte von Luft bei Normaldruck etwa drei Größenordnungen unter der Dichte von reiner Milch liegt, und der Volumenanteil der Luft in der Größenordnung von einigen wenigen % liegt, kann die Dichte von Milch mit Luft folgendermaßen abgeschätzt werden:

$$\rho_{Milch\ mit\ Luft} = \rho_{Milch}(1 - a_{Luft}) + \rho_g\alpha$$

$$\rho_{Milch\ mit\ Luft} \approx \rho_{Milch}(1 - a_{Luft}) \qquad (M2)$$

Damit kann die Gleichung C1 für die Schallgeschwindigkeit geschrieben werden als:

$$c = \left[\frac{a_{Luft}}{c_{Luft}^2} + \frac{(1 - a_{Luft})^2}{c_{Milch}^2} + \frac{a_{Luft}\rho_{Milch\ mit\ Luft}}{\gamma p}\right]^{-\frac{1}{2}}$$

Unter Vernachlässigung der quadratischen Terme in $a_{Luft}$ wird daraus:

$$c = \left[\frac{a_{Luft}}{c_{Luft}^2} + \frac{1 - 2a_{Luft}}{c_{Milch}^2} + \frac{a_{Luft}\rho_{Milch\ mit\ Luft}}{\gamma p}\right]^{-\frac{1}{2}}$$

Umstellung nach $a_{Luft}$ ergibt für den Luftvolumenanteil einen Wert von

$$a_{Luft} = \frac{\frac{1}{c_{Milch\ mit\ Luft}^2} - \frac{1}{c_{Milch}^2}}{\frac{1}{c_{Milch\ mit\ Luft}^2} - \frac{2}{c_{Milch}^2} + \frac{\rho_{Milch\ mit\ Luft}}{\gamma p}}$$

Tatsächlich wird der Nenner über den für die Milchverarbeitung relevanten Druckbereich im Wesentlichen durch den dritten Term dominiert, so dass mit der folgenden Näherung gearbeitet werden kann:

$$a_{Luft} \approx \frac{\gamma p}{\rho_{Milch\ mit\ Luft}} \cdot \left(\frac{1}{c_{Milch\ mit\ Luft}^2} - \frac{1}{c_{Milch}^2}\right) \qquad (A1)$$

Für die Schallgeschwindigkeit $c_{Milch}$ in reiner Milch ohne Luft ist hier ein Referenzwert zu setzen.

Wie in Fig. 2c dargestellt, wird zur Bestimmung des Luftanteils in Schritt 1141 ein Druckwert der mit Gas beladenen Flüssigkeit ermittelt, welcher in der Milch zum Zeitpunkt der Messung der Eigenfrequenzen $f_1$ und $f_3$ herrscht, denn auf deren Basis wurden mit Gleichung M1 die Dichte $\rho_{Milch\ mit\ Luft}$ sowie mit Gleichung C1 die Schallgeschwindigkeit der mit Luft beladenen Milch $c_{Milch\ mit\ Luft}$ ermittelt.

Für den Adiabatenkoeffizienten $\gamma$ gilt:
$\gamma = c_p/c_v = (f + 2)/f$, wobei f die Zahl der molekularen Freiheitsgrade des Gases ist, beträgt bei Raumtemperatur beispielsweise für Stickstoff und trockene Luft 1,4.

In einem Schritt 1142 wird dann auf Basis des Druckmesswerts, der zuvor ermittelten Dichte der mit Luft beladenen Milch $\rho_{Milch\ mit\ Luft}$ sowie der zuvor ermittelten Schallgeschwindigkeit der mit Luft beladenen Milch $c_{Milch\ mit\ Luft}$ mit Gleichung A1 der Luftvolumenanteil $a_{Luft}$ berechnet.

In einem Schritt 1143 folgt die Berechnung der gesuchten Dichte $\rho_{Milch}$ für die von der Luft befreiten Milch folgt:

$$\rho_{Milch} \approx \rho_{Milch\ mit\ Luft} \frac{1}{1-a_{Luft}} \qquad (M3)$$

Damit ist die erste Messgröße gefunden, um das Gleichungssystem G1, G2, G3 zu lösen.

Im Folgenden wird der zweite Teilprozess 120 beschrieben, in welchem die zweite Messgröße, nämlich die relative Permittivität ermittelt wird.

Grundlage hierfür ist eine Messung 121 der Ausbreitungseigenschaften einer elektromagnetischen Welle (Amplitude und Phase des empfangenen Signals in Bezug auf das ausgesendete Signal) innerhalb des Mediums in der Rohrleitung zwischen einer Sendeantenne und einer Empfangsantenne mit Abstand d. Diese Messung 121 kann mit elektromagnetischen Wellen unterschiedlicher Frequenz f durchgeführt werden, so dass eine Übertragungsfunktion im Frequenzbereich S(f) innerhalb eines Bandes von z.B. 2 GHz - 4 GHz ermittelt wird 122.

In praktischen Messsystemen enthält das gemessene Spektrum S(f) nicht ausschließlich die (vom Medium abhängigen) Ausbreitungseigenschaften auf der Strecke zwischen Sende- und Empfangsantenne, sondern zusätzlich auch die Dämpfung und Phasendrehung der Antennen, Anschlusskabel sowie Übergangsstellen. Hinzu kommen ggfs. noch Einflüsse durch mehrfache Reflektionen im Bereich der Anschlusskabel. Durch geeignete Referenzmessungen können diese Einflüsse weitgehend charakterisiert und in der Folge Messungen kompensiert 123 werden, so dass nur der relevante Teil der Übertragungsfunktion zwischen Sende- und Empfangsantenne verbleibt.

Von der Übertragungsfunktion S(f) kann durch inverse Fourier-Transformation auf die Impulsantwort im Zeitbereich zurückgerechnet werden 124. Durch die Messung eines begrenzten Bandbereiches handelt es sich dabei dann auch um die Impulsantwort des Systems auf Anregung mit einem bandbegrenzten Impuls, dessen Form sich aus der Form der vor der inversen Fourier-Transformation angewendeten Fensterfunktion ergibt. Aus der Position des Maximums dieses verzögerten Impulses bezüglich der Zeitachse lässt sich die Gruppenlaufzeit $\tau_g$ innerhalb des vermessenen, bandbegrenzten Bereiches ermitteln 125. Aus dieser Größe lässt sich auf einfachem Weg die Ausbreitungsgeschwindigkeit des Signals abschätzen 126:

$$c = \frac{d}{\tau_g}$$

In vielen polaren Medien tritt Dispersion auf (Abhängigkeit der Permittivität und damit der Ausbreitungsgeschwindigkeit von der Frequenz der elektromagnetischen Welle). Aus diesem Grund ist die zuvor abgeschätzte, mittlere Laufzeit im Sinne einer Gruppenlaufzeit zur direkten Bestimmung der Medieneigenschaften nur eingeschränkt geeignet. Um eine präzise Messung zu ermöglichen kann der Phasenverlauf $\varphi = \arg(S(f))$ genutzt werden, indem in dem gemessenen Frequenzband die Phasenlaufzeit in Abhängigkeit der Frequenz berechnet wird 127:

$$\tau_{ph}(f) = -\frac{d\varphi(f)}{df}$$

Die Mehrdeutigkeit des Phasenverlaufes, beschreibbar durch ein ganzzahliges n in $\varphi_{real} = \varphi_{gemessen} + n \cdot 2\pi$, lässt sich beseitigen, indem dasjenige n gewählt wird, für das die Abweichung zwischen $\tau_{ph}$ und $\tau_{gr}$ minimal wird. Damit ist nun der Phasenverlauf und durch

$$c(f) = \frac{d}{\tau_{ph}(f)}$$

damit auch der genaue Verlauf der Ausbreitungsgeschwindigkeit über der Frequenz bestimmbar 128. Der Verlauf der Dämpfung d ist direkt aus dem Amplitudenverlauf von S(f) bekannt.

Aus den nun bekannten Verläufen von c und $\alpha$ kann durch Lösen der beiden folgenden Gleichungen 129 direkt umgerechnet werden in die physikalische Größe der komplexwertigen Permittivität des Mediums $\epsilon^* = \epsilon' + j\,\epsilon''$:

$$c = \left[\frac{\mu\epsilon_0\epsilon'}{2}\left[\sqrt{1 + \left(\frac{\epsilon''}{\epsilon'}\right)^2} + 1\right]\right]^{-\frac{1}{2}}$$

$$\alpha = \omega\left[\frac{\mu\epsilon_0\epsilon'}{2}\left[\sqrt{1 + \left(\frac{\epsilon''}{\epsilon'}\right)^2} - 1\right]\right]^{\frac{1}{2}}$$

$\omega$: Kreisfrequenz (w = $2\pi$f)
$\mu$: Permeabilität, $\mu = \mu_0\mu_r$

$\mu_0$: Magnetische Feldkonstante,

$$\mu_0 = 4\pi \cdot 10^{-7}\frac{N}{A^2}$$

$\mu_r$: Relative Permeabilität

$\epsilon_0$: Elektrische Feldkonstante,

$$\epsilon_0 \approx 8{,}854 \cdot 10^{-12}\frac{As}{Vm}$$

Die aus der Messung bestimmten Werte für $\epsilon^*$ oder $\epsilon'$ können nun in Gleichung G2 genutzt werden, entweder durch Herausgreifen des Wertes an einer zuvor definierten Messfrequenz oder durch Verarbeiten des gesamten Messdatenvektors in G2.

Damit sind die Voraussetzungen gegeben, um das Gleichungssystem G1, G2, G3 zu lösen und somit die Anteile der Komponenten in der Milch zu bestimmen, insbesondere den Fettanteil.

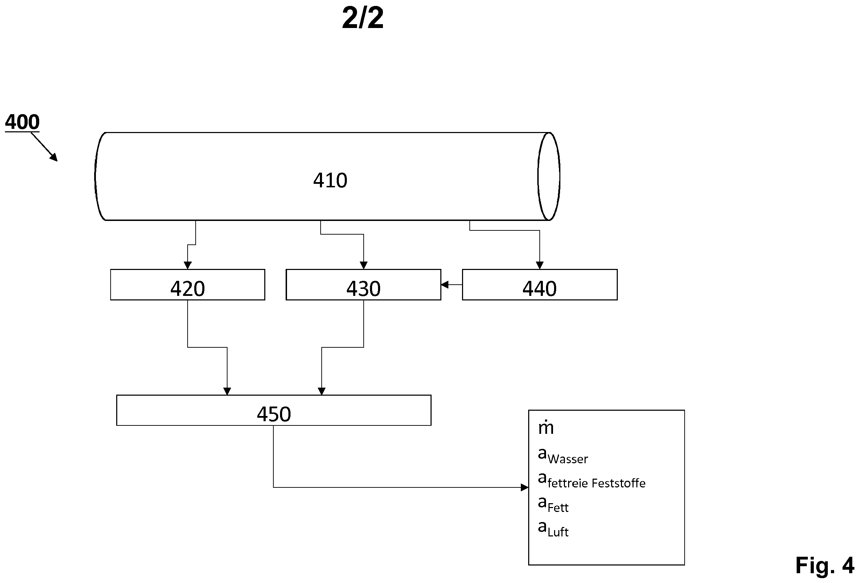

Fig. 4 zeigt schließlich eine erfindungsgemäße Messanordnung 400 zur Bestimmung des Milchfettanteils, insbesondere mittels des erfindungsgemäßen Verfahrens. Die Messanordnung 400 umfasst in eine Rohrleitung 400 eingebaute Messgeräte, nämlich einen Mikrowellensensor 420, einen Coriolis- Massedurchflussmesser 430, zum Erfassen der Dichte und des Massedurchflusses eines in der Rohrleitung 410 strömenden Mediums, insbesondere einen Coriolis-Massedurchflussmesser mit zwei in der Ruhelage gebogenen Messrohren, sowie einen Absolutdrucksensor 440, welcher einen Messwertausgang aufweist, der an einen Hilfssignaleingang des Coriolis-Massedurchflussmessers angeschlossen ist. Die Messanordnung 4000 umfasst weiterhin eine Rechnereinheit 450, welche an die Signalausgänge des Mikrowellensensors 420 und des Coriolis- Massedurchflussmessers 430 angeschlossen ist. Der Mikrowellensensor 420 ist dazu eingerichtet anhand von Signallaufzeiten, Permittivitätswerte und/oder die Absorption des in der Rohrleitung strömenden Mediums zu erfassen und an die Rechnereinheit 450 auszugeben. Der Coriolis- Massedurchflussmesser 430 ist dazu eingerichtet, neben dem Massedurchfluss m die Dichte, den Luftanteil $a_{Luft}$ und die Medientemperatur T zu ermitteln und an die Rechnereinheit 450 auszugeben. Die Rechnereinheit 450 ist dazu eingerichtet auf Basis dieser Eingangsgrößen die Zusammensetzung des in der Rohrleitung strömenden Mediums zu ermitteln und auszugeben unter der Annahme, dass es sich bei dem Medium um Milch handelt.

**Patentansprüche**

1. Verfahren zum kontinuierlichen Bestimmen des Fettgehaltes von Milch mit veränderlichen Feststoffanteilen welche mit veränderlicher Gasbeladung in einer Rohrleitung (410) fließt, umfassend:

   Ermitteln eines Wertes für die Schallgeschwindigkeit und eines mittleren Dichtewertes für die in der Rohrleitung (410) strömende Milch auf Basis der Eigenfrequenzen mindestens zweier Biegeschwingungsnutzmoden von Messrohren eines in der Rohrleitung (410) angeordneten Dichtemessers;

Ermitteln eines Werts für den statischen Druck in der Rohrleitung (410) mittels eines an die Rohrleitung (410) angeschlossenen Drucksensors (440);

Ermitteln eines Werts für den Gasvolumenanteil auf Basis des Werts für die Schallgeschwindigkeit, des Werts für die mittlere Dichte und des Werts für den Druck;

Ermitteln eines Werts der Dichte der in der Rohrleitung (410) strömenden Milch ohne die Gasbeladung auf Basis des Werts für die mittlere Dichte und auf Basis des Werts für den Gasvolumenanteil;

Ermitteln eines Werts für die Permittivität der in der Rohrleitung (410) strömenden Milch auf Basis von mindestens einer Messung der Ausbreitungsgeschwindigkeit und/oder Absorption von Mikrowellen in der Milch mittels eines in der Rohrleitung (410) angeordneten Mikrowellensensors (420); und

Berechnen des Fettanteils auf Basis des Werts der Dichte der in der Rohrleitung (410) strömenden Milch ohne die Gasbeladung und des Werts für die effektive Permittivität.

2. Verfahren nach Anspruch 1, wobei bei der Berechnung die Milch als Dreikomponentensystem modelliert wird wobei die Komponenten Fett, Wasser und fettfreie Feststoffe umfassen.

3. Verfahren nach Anspruch 2, wobei die Feststoffe Proteine und Kohlenhydrate, insbesondere hauptsächlich Lactose umfassen.

4. Verfahren nach einem der vorhergehenden Ansprüche, wobei die Dichte der in der Rohrleitung (410) strömenden Milch ohne die Gasbeladung als Funktion, beispielsweise als lineare Funktion der Konzentration der in der Milch enthaltenen Komponenten modelliert ist mit den Dichtewerten der reinen Komponenten als Gewichtungsfaktoren;

wobei die effektive Permittivität der in der Rohrleitung (410) strömenden Milch unter Berücksichtigung der Gasbeladung als Funktion der Konzentration der in der Milch enthaltenen Komponenten und der Permittivitätswerte der reinen Komponenten modelliert wird; und

wobei die Konzentration der Komponenten ermittelt wird, die zu den ermittelten Werten der Dichte und der effektiven Permittivität der Milch führen.

5. Verfahren nach einem der vorhergehenden Ansprüche, wobei die Bestimmung der Permittivität bei mindestens einer Frequenz oberhalb von 1 GHz, insbesondere oberhalb von 2 GHz, beispielsweise bei 2,45 GHz erfolgt.

6. Verfahren nach einem der vorhergehenden Ansprüche, weiterhin umfassend:

Messen der Temperatur der in der Rohrleitung (410) strömenden Milch; und

Ermitteln von temperaturabhängigen Werten für die Dichte und/oder Permittivität der in der Milch enthaltenen Komponenten.

7. Verfahren nach einem der vorhergehenden Ansprüche, wobei der Dichtemesser ein Coriolis-Massedurchflussmessgerät (430) umfasst, wobei das Verfahren weiterhin umfasst:

Ermitteln des Massedurchflusses, des Volumendurchflusses und/oder des Fettdurchflusses und/oder des Feststoffdurchflusses und/oder des fettfreien Feststoffdurchflusses und/oder des Wasserdurchflusses in der Rohrleitung (410).

8. Messanordnung (400) eingerichtet zum Bestimmen des Fettgehaltes von Milch in einer Rohrleitung (410) mit dem Verfahren nach einem der vorhergehenden Ansprüche, umfassend:

einen Dichtemesser mit mindestens einem schwingfähigen Messrohr, eingerichtet zum Ermitteln eines Dichtemesswertes und eines Schallgeschwindigkeitsmesswertes eines in dem Messrohr enthaltenen Mediums auf Basis mindestens von Nutzmodeeigenfrequenzen mindestens zweier Biegeschwingungsnutzmoden;

einen Drucksensor (440), eingerichtet zum Messen eines absoluten Drucks eines Mediums;

einen Mikrowellensensor (420), eingerichtet zum Ermitteln der Absorption und/oder Ausbreitungsgeschwindigkeit von Mikrowellensignalen in einem Medium;

und eine Rechnereinheit (450), eingerichtet zum Berechnen des Fettgehaltes auf Basis der Messwerte des Dichtemessers, des Drucksensors (440) und des Mikrowellensensors (420).

9. Messanordnung (400) nach Anspruch 8, außerdem umfassend:

- die Rohrleitung (410),

wobei der Dichtemesser, der Drucksensor (440) und der Mikrowellensensor (420) in die Rohrleitung (410) eingebaut sind.

10. Messanordnung (400) nach Anspruch 8 oder 9, wobei der Dichtemesser einen Coriolis-Massedurchflussmesser (430) umfasst.

## Claims

1. Method for the continuous determination of the fat content of milk with varying solids concentrations, wherein said milk flows through a pipe (410) with a variable gas load, wherein said method comprises the following steps:

   Determination of a value for the sound velocity and an average density value for the milk flowing in the pipe (410) on the basis of the natural frequencies of at least two flexural vibration useful modes of measuring tubes of a density meter arranged in the pipe (410);
   Determination of a value for the static pressure in the pipe (410) using a pressure sensor (440) connected to the pipe (410);
   Determination of a value for the gas volume content on the basis of the value of the sound velocity, the value for the average density and the value for the pressure;
   Determination of a value for the density of the milk flowing in the pipe (410) without the gas load on the basis of the value for the average density and on the basis of the value for the gas volume content;
   Determination of a value for the permittivity of the milk flowing in the pipe (410) on the basis of at least a measurement of the propagation speed and/or the absorption of microwaves in the milk by means of a microwave sensor (420) arranged in the pipe (410); and
   Calculation of the fat content on the basis of the value of the density of the milk flowing in the pipe (410) without the gas load and of the value of the effective perm ittivity.

2. Method as claimed in Claim 1, wherein, for the calculation, the milk is modelled as a three-component system, wherein the components comprise the materials fat, water and fat-free solids.

3. Method as claimed in Claim 2, wherein the solid materials comprise proteins and carbohydrates, particularly primarily lactose.

4. Method as claimed in one of the previous claims, wherein the density of the milk flowing in the pipe (410) without the gas load is modelled as a function, for example as a linear function, of the concentration of the components contained in the milk, with the density values of the pure components as weighting factors;

   wherein the effective permittivity of the milk flowing in the pipe (410) is modelled, taking the gas load into account, as a function of the concentration of the components contained in the milk and of the permittivity values of the pure components; and
   wherein the concentration of the components that lead to the values determined for the density and the effective permittivity of the milk are determined.

5. Method as claimed in one of the previous claims, wherein the permittivity is at least determined at a frequency higher than 1 GHz, particularly higher than 2 GHz, for example 2.45 GHz.

6. Method as claimed in one of the previous claims, further comprising the following steps:

   Measurement of the temperature of the milk flowing in the pipe (410); and
   Determination of temperature-dependent values for the density and/or permittivity of the components contained in the milk.

7. Method as claimed in one of the previous claims, wherein the density meter comprises a Coriolis mass flowmeter (430), said method further comprising the following step:
   Determination of the mass flow, the volume flow and/or the flow of fatty materials and/or the flow of solid materials and/or the flow of fat-free solid materials and/or the flow of water in the pipe (410).

8. Measuring arrangement (400) designed to determine the fat content of milk in a pipe (410) with the method as claimed in one of the previous claims, said arrangement comprising:

a density meter with at least a measuring tube that is able to vibrate, wherein said density meter is configured to determine a density measured value and a sound velocity measured value of a medium contained in the measuring tube on the basis of at least useful mode natural frequencies of at least two flexural vibration useful modes;
a pressure sensor (440) configured to measure an absolute pressure of a medium;
a microwave sensor (420) configured to determine the absorption and/or the propagation speed of microwave signals in a medium;
and a calculator unit (450), which is configured to calculate the fat content on the basis of the measured values of the density meter, the pressure sensor (440) and the microwave sensor (420).

9. Measuring arrangement (400) as claimed in Claim 8, further comprising:

- the pipe (410),
wherein the density meter, the pressure sensor (440) and the microwave sensor(420)
are installed in the pipe (410).

10. Measuring arrangement (400) as claimed in Claim 8 or 9, wherein the density meter comprises a Coriolis mass flowmeter (430).


**Revendications**

1. Procédé destiné à la détermination en continu de la teneur en matières grasses du lait à teneur variable en matières solides, lait qui s'écoule dans une conduite (410) avec une charge de gaz variable, lequel procédé comprend les étapes suivantes :

Détermination d'une valeur pour la vitesse du son et d'une valeur de densité moyenne pour le lait s'écoulant dans la conduite (410) sur la base des fréquences propres d'au moins deux modes utiles de vibration de flexion de tubes de mesure d'un densimètre disposé dans la conduite (410) ;
Détermination d'une valeur pour la pression statique dans la conduite (410) au moyen d'un capteur de pression (440) raccordé à la conduite (410) ;
Détermination d'une valeur pour la part de volume de gaz sur la base de la valeur de la vitesse du son, de la valeur de la densité moyenne et de la valeur de la pression ;
Détermination d'une valeur pour la densité du lait s'écoulant dans la conduite (410) sans la charge de gaz, sur la base de la valeur de la densité moyenne et sur la base de la valeur de la part de volume de gaz ;
Détermination d'une valeur pour la permittivité du lait s'écoulant dans la conduite (410) sur la base d'au moins une mesure de la vitesse de propagation et/ou de l'absorption des micro-ondes dans le lait au moyen d'un capteur à micro-ondes (420) disposé dans la conduite (410) ; et
Calcul de la part de matières grasses sur la base de la valeur de la densité du lait s'écoulant dans la conduite (410) sans la charge de gaz et de la valeur de la permittivité effective.

2. Procédé selon la revendication 1, pour lequel, lors du calcul, le lait est modélisé en tant que système à trois composants, les composants comprenant les matières grasses, l'eau et les matières solides non grasses.

3. Procédé selon la revendication 2, pour lequel les matières solides comprennent des protéines et des hydrates de carbone, notamment principalement du lactose.

4. Procédé selon l'une des revendications précédentes, pour lequel la densité du lait s'écoulant dans la conduite (410) sans la charge de gaz est modélisée en tant que fonction, par exemple en tant que fonction linéaire, de la concentration des composants contenus dans le lait, avec les valeurs de densité des composants purs comme facteurs de pondération ;

la permittivité effective du lait s'écoulant dans la conduite (410) étant modélisée, en tenant compte de la charge de gaz, en tant que fonction de la concentration des composants contenus dans le lait et des valeurs de permittivité des composants purs ; et
la concentration des composants - laquelle concentration conduit aux valeurs déterminées de la densité et de la permittivité effective du lait - étant déterminée.

5.  Procédé selon l'une des revendications précédentes, pour lequel la détermination de la permittivité est effectuée à au moins une fréquence supérieure à 1 GHz, notamment supérieure à 2 GHz, par exemple à 2,45 GHz.

6.  Procédé selon l'une des revendications précédentes, comprenant en outre les étapes suivantes :

    Mesure de la température du lait s'écoulant dans la conduite (410) ; et
    Détermination des valeurs de densité et/ou de permittivité des composants contenus dans le lait en fonction de la température.

7.  Procédé selon l'une des revendications précédentes, pour lequel le densimètre comprend un débitmètre massique Coriolis (430), le procédé comprenant en outre l'étape suivante :
    Détermination du débit massique, du débit volumique et/ou du débit de matières grasses et/ou du débit de matières solides et/ou du débit de matières solides non grasses et/ou du débit d'eau dans la conduite (410).

8.  Dispositif de mesure (400) conçu pour la détermination de la teneur en matières grasses du lait dans une conduite (410) avec le procédé selon l'une des revendications précédentes, lequel dispositif comprend :

    un densimètre comprenant au moins un tube de mesure apte à vibrer, lequel densimètre est conçu pour déterminer une valeur mesurée de densité et une valeur mesurée de vitesse du son d'un produit contenu dans le tube de mesure sur la base d'au moins des fréquences propres de mode utile d'au moins deux modes utiles de vibration de flexion ;
    un capteur de pression (440) conçu pour mesurer une pression absolue d'un produit ;
    un capteur à micro-ondes (420) conçu pour déterminer l'absorption et/ou la vitesse de propagation de signaux micro-ondes dans un produit ;
    et une unité de calcul (450), laquelle est conçue pour calculer la teneur en matières grasses sur la base des valeurs mesurées par le densimètre, le capteur de pression (440) et le capteur à micro-ondes (420).

9.  Dispositif de mesure (400) selon la revendication 8, comprenant en outre :

    - la conduite (410),

    le densimètre, le capteur de pression (440) et le capteur à micro-ondes (420) étant montés dans la conduite (410).

10. Dispositif de mesure (400) selon la revendication 8 ou 9, pour lequel le densimètre comprend un débitmètre massique Coriolis (430).

**100**

| 110 |
|-----|

↓

| 120 |
|-----|

↓

| 130 |
|-----|

**Fig. 1**

---

**110**

| 111 |
|-----|

↓

| 112 |
|-----|

↓

| 113 |
|-----|

↓

| 114 |
|-----|

**Fig. 2a**

---

**113**

| 1131 |
|------|

↓

| 1132 |
|------|

↓

| 1133 |
|------|

↓

| 1134 |
|------|

↓

| 1135 |
|------|

**Fig. 2b**

---

**114**

| 1141 |
|------|

↓

| 1142 |
|------|

↓

| 1143 |
|------|

**Fig. 2c**

---

**120**

| 121 |
|-----|

↓

| 122 |
|-----|

↓

| 123 |
|-----|

↓

| 124 |
|-----|

↓

| 125 |
|-----|

| 126 |
|-----|

↓

| 127 |
|-----|

↓

| 128 |
|-----|

↓

| 129 |
|-----|

**Fig. 3**

EP 3 729 074 B1

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7363800 B2

- US 5103181 A